(19) Europäisches Patentamt
European Patent Office
Office européen des brevets

(11) **EP 2 649 935 A2**

(12) **EUROPEAN PATENT APPLICATION**

(43) Date of publication:
**16.10.2013 Bulletin 2013/42**

(51) Int Cl.:
*A61B 5/0484* (2006.01)

(21) Application number: **13162286.2**

(22) Date of filing: **04.04.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **12.04.2012 JP 2012091279**

(71) Applicant: **Canon Kabushiki Kaisha Tokyo 146-8501 (JP)**

(72) Inventors:
• **Miyazaki, Jungo**
  **Tokyo, Tokyo 146-8501 (JP)**
• **Ichimura, Yoshikatsu**
  **Tokyo, Tokyo 146-8501 (JP)**
• **Otsu, Ryuichi**
  **Tokyo,Tokyo 146-8501 (JP)**

(74) Representative: **TBK Bavariaring 4-6 80336 München (DE)**

(54) **Evaluation method, evaluation device, program, and recording medium**

(57)    An information acquiring method configured to acquire brain activity information from biological subjects (502) presented a sensory stimulus, in which the brain activity information is acquired by measuring the brain activity in at least one region of the brain areas of the biological subjects including the visual cortices of the cerebral cortex, the auditory cortices of the cerebral cortex, and the vestibular cortices of the cerebral cortex; while the stimulus which changes over time is presented to the brain areas by stimulus presentation means (503, 505) before and after the sensory stimulus is presented to the brain areas.

## FIG. 5

EP 2 649 935 A2

**Description**

BACKGROUND OF THE INVENTION

Field of the Invention

The present technology relates to an information acquiring method, information acquiring device, information acquiring program and recording medium for acquiring brain activity information from biological subjects given a sensory stimulus.

Description of the Related Art

Watching video filmed on commercial video cameras with shakiness due to hand movement on a large screen is known to cause a feeling of discomfort similar to motion sickness in some viewers (so-called visually induced motion sickness). Also, watching 3D images is known to cause a feeling of discomfort similar to visually induced motion sickness, which is called 3D motion sickness. The specific biological reactions caused by visually induced motion sickness and 3D motion sickness include nausea, dizziness, and disorientation.

According to "Cerebral blood flow and subjective  score during visually induced motion sickness.", Iijima, Atsuhiko, et al., 2008, 23rd BPES Symposium on Biological and Physiological Engineering, pp. 11-12, the method for evaluating visually induced motion sickness involves measuring changes in cerebral blood flow of subjects while watching video. Specifically, a 20-minute video which includes shakiness due to hand movement and swinging of the camera was shown to the subjects, and the changes in cerebral blood flow of the subjects was measured using a Near Infrared Spectroscopy (NIRS) system (NIRO-200, Hamamatsu Photonics) wherein the relationship between the video and changes in cerebral blood flow was analyzed.

The inventors of the present technology have uncovered a problem based on the method disclosed in "Cerebral blood flow and subjective score during visually induced motion sickness." That is to say, according to the method in "Cerebral blood flow and subjective score during visually induced motion sickness.", changes in cerebral blood flow were measured in the frontal cortex, which carry out executive functions such as reasoning and decision making. This makes it difficult to determine whether these changes in cerebral blood flow are due to the viewing of the video, or due to some other cause. For this reason, a  method has been needed that could acquire information related to a stress similar to that of visually induced motion sickness so as to increase accuracy thereof.

SUMMARY OF THE INVENTION

The present invention in its first aspect provides an information acquiring method as specified in claims 1 to 9.

The present invention in its second aspect provides an information acquiring device as specified in claims 10 to 18.

The present invention in its third aspect provides an information acquiring program as specified in claim 19.

The present invention in its fourth aspect provides a recording medium as specified in claim 20.

The present invention in its fifth aspect provides an information acquiring program as specified in claim 21.

The present invention in its fourth aspect provides a recording medium as specified in claim 22.

Further features of the present invention will  become apparent from the following description of exemplary embodiments with reference to the attached drawings.

BRIEF DESCRIPTION OF THE DRAWINGS

Fig. 1 is a diagram describing an information acquiring method according to an embodiment of the present technology.

Fig. 2 is a diagram describing the visual cortex.

Fig. 3 is a diagram illustrating the places to position electrodes according to the International 10-20 System.

Fig. 4 is a diagram illustrating an expression for calculating (a) a coherence and (b) a cross correlation.

Fig. 5 is a diagram describing an information acquiring device according to an embodiment of the present technology.

Fig. 6 is a diagram describing a functional magnetic resonance imaging device used for measuring cerebral blood flow according to an embodiment in the present technology.

Fig. 7 is a diagram describing a stimulus which changes over time used in an embodiment of the present technology.

Fig. 8 is a diagram illustrating one example of brain activity data acquired by an embodiment of the present technology.

Fig. 9 is a table illustrating the coherences calculated in an embodiment of the present technology.

DESCRIPTION OF THE EMBODIMENTS

The following describes an embodiment of the present technology, but the present technology is not limited thusly. First, the information acquiring method according to the present embodiment will be described.

The information acquiring method according to the present embodiment is configured to acquire brain activity information from biological subjects given a sensory stimulus, characterized by the inclusion of the following processes (1) through (6):

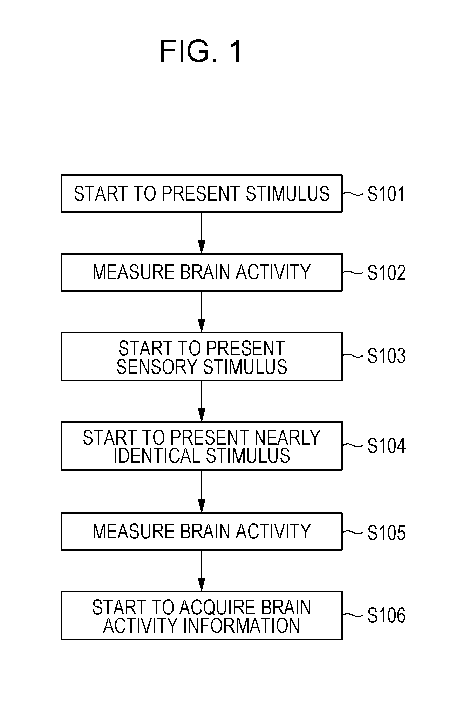

(1) As illustrated by step S101 in Fig. 1, this starts the presentation of a stimulus which changes over time to at least one region of the brain areas of the biological subjects including the visual cortices (hereafter, the visual cortices), the auditory cortices (hereafter, the auditory cortices), and the vestibular cortices (hereafter, the vestibular cortices).

(2) As illustrated by step S102 in Fig. 1, this measures brain activity in at least one region in the brain areas while the stimulus which changes over time is presented.

(3) As illustrated by step S103 in Fig. 1, this starts the presentation of the sensory stimulus after the process (2).

(4) As illustrated by step S104 in Fig. 1, this starts the presentation of a nearly identical stimulus to the stimulus which changes over time to the brain areas after the process (3).

(5) As illustrated by step S105 in Fig. 1, this starts the measurement of brain activity for at least one region of the brain areas when the nearly identical stimulus is presented.

(6) As illustrated by step S106 in Fig. 1, this starts the acquirement of the brain activity information on the basis of the brain activity data measured in the process (2) and the brain activity data measured in the process (5).

Processes (1) and (2)

When a stimulus which changes over time is presented to any of the brain areas of biological subjects including the visual cortices, the auditory cortices, and the vestibular cortices, neurons in the brain area that received input from the sensory organ that received the stimulus are activated. For example, when a light stimulus which changes over time is presented to a biological subject, the visual cortices are activated in response to the presented light stimulus. Further, the brain activity may be quantified by measuring cerebral blood flow, action potential of nerve cells, and the electromagnetic field generated thereby. The brain is activated in response to the stimulus which changes over time presented to a biological subject. For example, when reciprocally repeating a strong stimulus and a weak stimulus, the brain activity reciprocally repeats between an active state and an inactive state. That is to say, the action potential reciprocally repeats between an active state and an inactive state. As the action potential reciprocally changes between this active state and inactive state, cerebral blood flow reciprocally repeats between a large flow state and small flow state.

Process (3)

After the process (2), a sensory stimulus is presented to the brain area to which the stimulus which changes over time was presented. The presentation of a sensory stimulus is, for example, the presenting of a video.

Processes (4) and (5)

After the process (3), a nearly identical stimulus to that of the stimulus which changes over time used in process (2) is presented to the brain area to which the stimulus was presented in the process (2), and the brain activity of at least one region of the brain areas is measured at this time.

Process (6)

Brain activity information is acquired from the biological subject subjected to sense stimulus on the basis of the brain activity data measured in the process (2) and the brain activity data measured in the process (5). Acquiring the brain activity information is, for example, acquiring information related to stress provided to a biological subject, using the correlation relationship of the brain activity data measured in the process (2) and the brain activity data measured in the process (5) as an index. When there is a strong correlation relationship between the brain activity data measured in the process (2) and the brain activity data measured in the process (5), the stress provided to the subjects by the sensory stimulus is weak, and when there is a weak correlation relationship, the provided stress may be evaluated as strong. The correlation relationship may be quantified by a coherence value and a cross correlation, which will be described later.

Using such a method to measure the brain activity of regions of brain areas which perform early processing of sensory information, i.e. the visual cortices, auditory cortices, and the vestibular cortices, rather than the brain areas such as the frontal cortex which governs executive functions, the brain activity information from biological subjects to which a sensory stimulus has been presented may be acquired, for example, such as information related to stress. For this reason, factors other than from the sensory stimulus is not likely to be present when acquiring information related to stress from the presentation of the sensory stimulus. Therefore, as factors other than the stimulus presented by the sensory stimulus to the subjects are not likely to have any influence, the evaluation method according to the present embodiment may be said to be highly accurate as a method for acquiring information, for example, as an acquiring method for stress from biological subjects to which a sensory stimulus has been presented.

The information acquiring method according to the present embodiment is preferred when the sensory stimulus is a video. That is to say, brain activity information from biological subjects to which video is presented is acquired, and this video may be evaluated from the acquired brain activity information. Therefore, this method may be said to be highly accurate as a video evaluation method. Sensory Stimulus

The sensory stimulus in the present embodiment is not particularly restricted as long as it causes changes in brain activity for any of the brain areas of subjects including the visual cortices, auditory cortices, and the vestibular cortices. Examples include a visual stimulus, such as a video, that changes brain activity in the visual cortex, an auditory stimulus, such as sound, that changes brain activity in the auditory cortex, an audio-visual stimulus, such as a video with sound, that changes brain activity in the visual cortex and the auditory cortex, and a sense of balance stimulus, such as the feeling of acceleration presented to subjects by a roller coaster, that changes brain activity in the vestibular cortices. The sensory stimulus may be any one of the visual stimulus, auditory stimulus, or the sense of balance stimulus, or may be combination of a multiple of these stimuli. A flight simulator, for example, simultaneously presents a visual stimulus and a sense of balance stimulus to a biological subject. When the sensory stimulus is a video, the evaluation method according to the present embodiment is preferably an evaluation method of video.

Biological Subjects

The biological subjects in the present embodiment indicate living organisms such as humans, monkeys, and dogs. Humans are the preferred subjects with respect to the information acquiring method according to the present embodiment. The following descriptions assume the biological subject to be human. Also, humans are sometimes referred to as subjects in the present specification.

Brain Activity Information

The brain activity in the present embodiment represents a brain response from biological subjects to which the previously described sensory stimulus is presented. The brain response may be quantified by measuring changes in action potential of nerve cells in the brain, changes in the electromagnetic field produced by changes in the action potential, and cerebral blood flow. The action potential of nerve cells in the brain may be measured using a nerve activity recording device that uses electrodes. The changes in the electromagnetic field produced by changes in the action potential may be measured by an electroencephalograph or a magnetoencephalograph. The changes in cerebral blood flow produced by changes in the action potential may be measured using a functional magnetic resonance imaging (fMRI) device, a NIRS device, or a positron emission tomography device. Among these, a method using a NIRS device is preferable due to the low cost of the device and that testing may be implemented safely without restricting subjects.

The brain activity information in the present embodiment represents information measurement data of the previously described brain activity and the information acquired from this data. For example, the brain activity information may be information related to stress. The information related to stress may be acquired from data regarding changes in action potential in nerve cells in the brain, changes in the electromagnetic field produced by changes in the action potential, and changes in cerebral blood flow.

Stress

The stress in the present embodiment represents functional changes in the mind and body of subjects when various external stimuli work as strain on subjects. The subjects may exhibit such physiological responses as tiredness in the eyes, headache, sweating, nausea, dizziness, disorientation, eye strain, abnormalities of the autonomic nervous system, and abnormalities of the sense of balance system. The information acquiring method according to the present embodiment preferably acquires information related to stress in biological subjects. Also, the information acquiring method according to the present embodiment may preferably be used when evaluating such stress as when causing biological responses such as visually induced motion sickness or 3D motion sickness. Visual Cortices

The sense of vision in the present embodiment is the sense in which visible light is physically input, and visual information refers to information regarding color, shape, movement, texture, and depth of physical objects in the outer world, information regarding categories of physical objects, and spatial information regarding locational relationship between physical objects. The brain area related to the vision is the group of visual cortices.

When the brain activity to be measured is that in the visual cortices using the evaluation method in the present embodiment, any brain region exhibiting neural response may be measured from among those in the visual cortices including the primary visual cortex (V1 (dorsal V1: V1d, ventral V1: V1v)), the secondary visual cortex (V2 (dorsal V2: V2d, ventral V2: V2v), the tertiary visual cortex (V3, V3 accessory: V3A), the fourth visual cortex (V4 (dorsal V4: V4d, ventral V4: V4v)), the middle temporal area (MT), the middle superior temporal area (MST), the seventh visual cortex (V7), the ventral posterior area (VP), the lateral occipital complex (LOc), and the eighth visual cortex (V8). Also, brain activity of regions spanning multiple brain areas (the region spanning V1 and V2, for example) in the visual cortices may be measured, or the brain activity in a region that is a portion of a brain area may be measured.

Further, there are theories stating other types of visual cortices equivalent to V4v and V8, and other types labeled V01 and V02. According to the present technology, these brain areas are specified with the labels V4v and V8. Among these regions, measuring the V3A, middle temporal area (MT), V7, and V4v, which process information relating to visual motion, binocular disparity, or shape, is preferable as this is thought to enable precise evaluation of stress causing biological responses such as visually induced motion sickness and 3D motion sickness.

Visual information is processed in the visual cortices, which are confirmed to be in the occipital lobe, a portion of the parietal lobe, and a portion of the temporal lobe. Further, the frontal cortex is positioned in the anterior of the cerebral cortex, the occipital lobe is positioned in the posterior, the parietal lobe is positioned at the top, and the temporal lobe is positioned on the side.

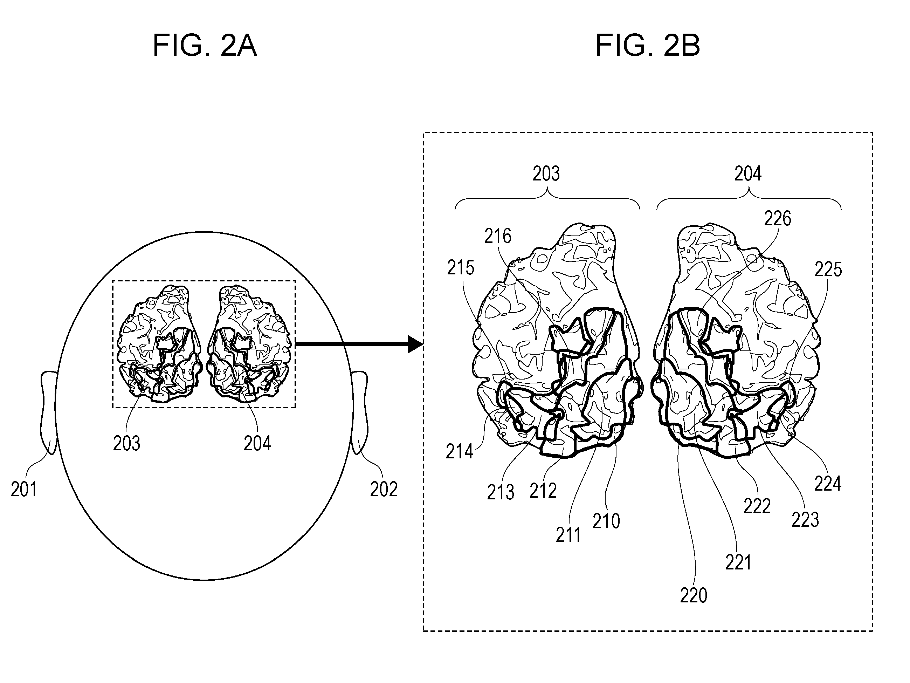

Fig. 2A is a schematic diagram of the surface of the cerebral cortex looked at from the occipital side. Fig. 2B is a magnified view of the surface of the cerebral cortex in Fig. 2A. Figs. 2A and 2B illustrate the cerebrum divided into left and right hemispheres for descriptive purposes, though the cerebrum is actually connected by the corpus callosum.

Fig. 2A illustrates a left ear 201, a right ear 202, a left cerebral hemisphere 203, and a right cerebral hemisphere 204. A V1 (V1v, V1d) 210, V2 (V2v, V2d) 211, V3 212, lateral occipital complex (Loc) 213, middle temporal area (MT) 214, middle superior temporal area (MST) 215, and V3A 216 are illustrated in the left cerebral hemisphere 203 (Fig. 2B). Also, a V1 (V1v, V1d) 220, V2 (V2v, V2d) 221, V3 222, lateral occipital complex (Loc) 223, middle temporal area (MT) 224, middle superior temporal area (MST) 225, and V3A 226 are illustrated in the right cerebral hemisphere 204 (Fig. 2B).

As illustrated in Fig. 2B, each visual cortex is positioned nearly symmetrically in both hemispheres. The visual information is first input toward the V1 in the posterior end of the occipital lobe in both the left and right hemispheres. Afterwards, information processing is performed in the visual cortices that govern executive functions such as V2, V3, and V3A. The visual information processed in the occipital visual cortices travels up through each visually related cortex of the parietal lobe, the temporal lobe, and the frontal lobe, where it is integrated to form such visual functions as visual perception and visual memory. Further, V1 is positioned at area 17, V2 is positioned at area 18, and V3 is positioned at area 19 on Brodmann's brain map.

Visual information processing after V1 and V2 are primarily processed in two paths within the visual cortices. The first is called the dorsal stream, and this visual information processing pathway goes through brain areas that extend into the posterior side such as V3, V3A, the middle temporal area (MT), the medial superior temporal area (MST), and the lateral occipital complex (Loc). Many nerve cells that respond to visual motion or binocular disparity exist in the dorsal stream, and are thought to be involved in recognition of one's position in space and motive state. The other is called the ventral stream, and this visual information processing pathway goes through brain areas that extend into ventral part such as ventral fourth visual cortex (V4v) and the eighth visual cortex (V8). Many nerve cells that respond to color and shape exist in the ventral stream, and are thought to be involved in recognition of visual object representation.

When visually induced motion sickness occurs, neural responses to visual stimulus in the visual cortices are modulated. Therefore, stress such as visually induced motion sickness and 3D motion sickness may be detected by indexing brain activity in the visual cortices.

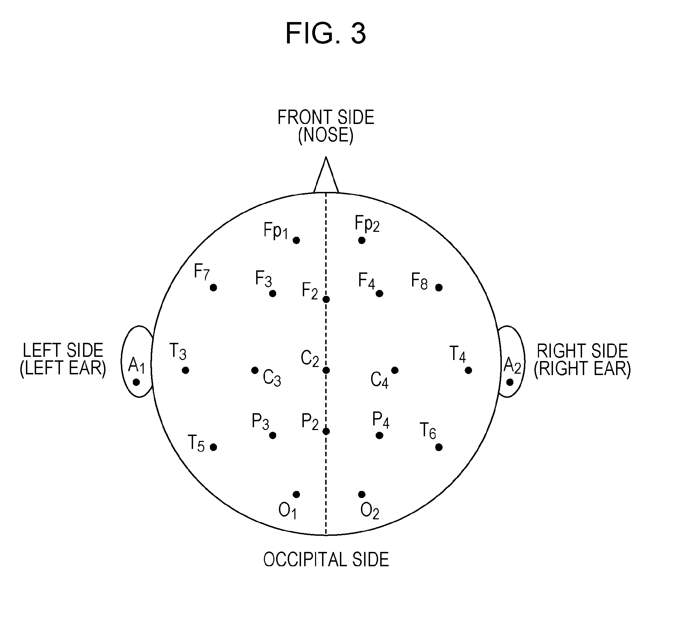

When measuring activity in the visual cortices with an electroencephalograph (EEG), the brain areas distributed at the entrance to the visual cortices such as V1, V2, and V3 are in the posterior side of the occipital lobe, and so signals may be detected from electrodes $O_1$ and $O_2$ as in the International 10-20 System. Also, the brain areas V3A and V7 are distributed at the parietal lobe side, and so it is thought that signals may be detected from electrodes $P_2$, $P_3$, and $P_4$ as in the International 10-20 System, and V4d, the middle temporal area (MT), the medial superior temporal area (MST), and the lateral occipital complex (Loc) are distributed at the temporal lobe side, so signal may be detected from electrodes $T_5$ and $T_6$ as in the International 10-20 System. The International 10-20 System is an international reference system that determines where to position measuring electrodes on the head when measuring brain activity by an electroencephalograph. Fig. 3 is a diagram illustrating places to position electrodes according to the International 10-20 System. For example, measuring brain activity for V1, V2, V3, etc. with an electroencephalograph may be performed by placing electrodes in positions $O_1$ and $O_2$ as in Fig. 3. Further, though it is preferable to determine electrode positions using the International 10-20 System, but electrode positions are not particular restricted.

Sense of Hearing, Auditory Cortices

The sense of hearing in the present embodiment is the sense in which sound is physical input, and audio information refers to strength of sound, pitch, timbre, sound source direction, rhythm, and pronunciation. The brain areas related to the sense of hearing are the auditory cortices. The auditory cortices include the primary, secondary, and tertiary auditory cortices, and are positioned in area 41 and area 42 on Brodmann's brain map. When measuring brain activity for the auditory cortices by an electroencephalograph, signals may be detected by electrodes $T_3$ and $T_4$ as in the International 10-20 System. Sense of Balance, Vestibular cortices

The sense of balance in the present embodiment is the sense in which information regarding the sense of balance (information on how far a person is leaning, moving or not, etc.) is physically input. The brain area related to the sense of balance is the vestibular cortices. According to the present embodiment, the vestibular cortices is defined as being positioned near the temporo-parietal junction, which is where the temporal and parietal lobes meet at the posterior end of the Sylvian fissure. When measuring brain activity for the vestibular cortices by an electroencephalograph, it is thought that signals may be detected by electrodes $T_3$, $T_4$, $P_3$, and $P_4$ as in the International 10-20 System.

The following describes an example using the visual cortices, but the auditory cortices and the vestibular cortices may also be measured in the same way. Regions for Measuring Brain Activity

The regions for measuring brain activity regarding the information acquiring method according to the present embodiment may be selected freely from at least any region of the brain areas including the previously described visual cortices, auditory cortices, and the vestibular cortices. For example, an arbitrary brain area within the visual cortices may be selected such as the middle temporal area (MT), or a region spanning multiple visual cortices may be selected such as the region spanning the middle temporal area (MT) and the middle superior temporal area (MST). Also, a region in a portion of a brain area for an arbitrary visual cortex may also be selected such as a region in a portion within the middle temporal area (MT). Further, when measuring brain activity in regions spanning multiple brain areas, the brain activity region in a portion of the multiple brain areas may be measured, such as a region of a portion of the middle temporal area (MT) and a region in a portion of the middle superior temporal area (MST). Though this description used the visual cortices as examples, the auditory cortices and the vestibular cortices are handled in the same way.

Stimulus Which Changes over Time

The stimulus which changes over time in the present embodiment continually or intermittently changes brain activity in any of the brain areas including the visual cortices, the auditory cortices, and the vestibular cortices. Also, the stimulus which changes over time may stimulate multiple types of brain areas including the visual cortices, the auditory cortices, and the vestibular cortices.

The proper stimulus which changes over time may be selected depending on the brain activity to be measured (action potential of nerve cells, the electromagnetic field produced thereby, or cerebral blood flow) and the measuring device (an electroencephalograph, a magnetoencephalograph, a nerve activity recording device using electrodes, a NIRS device, an fMRI device, or a positron emission tomography device).

When presenting the stimulus which changes over time to the visual cortex, stimuli which change over time may be used such as changes in moving and still visuals, changes in direction of movement in visuals, changes in speed, changes in brightness, changes in luminance, changes in color, shape, changes of objects, or any combination stimulus thereof.

When presenting stimulus which changes over time to the auditory cortex, stimuli which change over time may be used such as changes in intensity of sound, changes in pitch, changes in tone, changes in direction of sound source, changes in rhythm, or any combination stimulus thereof.

When presenting the stimulus which changes over time to the vestibular cortices, stimuli which change over time may be used such as changes in posture of subjects, that is to say, changes in movement or inclination of posture of subjects, state changes in the vestibular system caused artificially from the outside world, which specifically could be state changes caused by acceleration, running water or electricity from the outside world, or any combination stimulus thereof.

The stimulus which changes over time in the present embodiment preferably cause the brain response in the previously described brain areas to have temporal or frequency characteristics. For example, the stimulus may be at least two different types with different amounts of stimulus which are then reciprocally repeated. Also, the stimulus which changes over time could be any combination of multiple stimuli including visual stimuli, audio stimuli, or stimuli targeting the vestibular cortices. The stimuli with different amounts of stimulus may be presented continuously or intermittently over time.

Also, the stimulus which changes over time preferably is a visual stimulus of two types with different amounts of stimulus that are reciprocally repeated.

When a stimulus of two types with different amounts of stimulus is reciprocally repeated, the brain activity of the previously described brain areas repeatedly rise and fall in response to the change in the amount of stimulus, and represents a response having cyclical characteristics reflecting the cyclic repetition of the two types of stimulus. The response having cyclical characteristics may be readily analyzed using Fourier analysis, which enables the brain response characteristics to be quantitatively evaluated.

Examples of stimuli in which two types of stimulus with different amounts of stimulus are reciprocally repeated include video that reciprocally repeats movies and still images, an audio stimulus in which an arbitrary piece of music is reciprocally and repeatedly started and stopped, or a stimulus to the sense of balance in which the subject reciprocally and repeatedly changes from a moving state to a stopped state.

When using a stimulus in which two types of stimulus with different amounts of stimulus are reciprocally repeated, the preferable range of this repeating cycle (frequency) varies depending on the type of brain response to be measured, that is to say, whether to measure the action potential of nerve cell, the electromagnetic field produced thereby, or cerebral blood flow. Also, the preferable, applicable range of this repeating cycle(frequency) varies depending on the limitation on the temporal resolution of the measuring device used, which may be an electroencephalograph, a magnetoencephalograph, a nerve activity recording device using electrodes, a NIRS device, an fMRI device, or a positron emission tomography device. Also, the preferable, applicable range of this repeating cycle (frequency) also varies depending on the quality of the stimulus which changes over time. Here, the two types of different stimuli are counted together as one repetition, and the cycle is defined as a repeating cycle of at least two of the repetitions.

For example, when measuring cerebral blood flow and the change in cerebral blood flow as a result of receiving the stimulus exhibits a rise and fall response of 30 seconds, the cycle of the stimulus which changes over time is preferably at most 0.033 Hz.

Also, when using an fMRI device or a NIRS device, the temporal resolution of these devices is between one and three seconds, so the cycle of the stimulus which changes over time is preferably at most 0.3 Hz.

Also, when the stimulus which changes over time is the repetition between a state in which a light stimulus is presented and a state in which the light stimulus is not presented (hereafter, abbreviated as flicker), the cycle of the stimulus which changes over time is preferably at most 30 Hz from the limit of the subject being able to distinguish the flicker. Also, a cycle of at most 5 Hz is preferable as subjects are not likely to experience photosensitive epilepsy and brain activity is readily measured.

There are no particular upper limit restrictions on the repeating cycle (lower limit on the repeating frequency), but if the repeating cycle is too long (the repeating frequency is too low), the time to measure the brain response increases, and there is a potential of the load from the sensory stimulus to become a burden on the subjects, and so the repeating cycle between two and 30 seconds is preferable (repeating frequency between 0.03 and 0.5 Hz).

As a specific example of using a stimulus in which a visual stimulus of at least two types with reciprocally repeats at fixed time intervals with alternating amounts of stimulus, a video of an image with a black and white stripe pattern arranged in a radial fashion is repeatedly rotated and stopped at fixed time intervals. If the fixed time interval is 18 seconds, for example, the video may be repeated for a total of six repetitions of 18-second rotations and 18-second stills (216 total seconds). In this case, the repeating cycle is 36 seconds, that is to say, the repeating frequency is 1/36 Hz, or about 0.028 Hz. Further, the switching between the rotating image and the still image may be continuous or may be discontinuous. Brain Activity Measuring Method

Details on devices to measure brain activity will now be described. The electroencephalograph in the present embodiment measures electromagnetic fields produced by the action potential of nerve cells in the brain. Active electrodes are attached to the head of a subject, and time-series data between a reference electrode with an electrical potential of zero (normally an electrode attached to the earlobe) and changes in the electrical potential is measured.

Measuring brain waves is performed when the previously described stimulus which changes over time is presented. When using video that repeats between playing and stopping at fixed time intervals as the stimulus which changes over time, for example, the action potential of nerve cells in the visual cortices is evoked causing an increase in brain wave signals during playback of the video as compared to when the video is stopped. If playback and stopping of the video repeats at fixed time intervals, the brain wave signals in the visual cortices repeatedly increase and decrease in accompaniment with the playback and stopping. That is to say, if the repeating frequency of the playback and stopping of the stimulus which changes over time is designated as f, the waveform of the measured brain wave signals approximate the sine waveform of the frequency f.

When the stress provided to the subjects is small, the brain wave signals from the visual cortices of the subjects synchronously respond to the stimulus which changes over time, and signals of a waveform approximating the sine wave of the repeating frequency of the stimulus which changes over time may be acquired. In contrast, when the stress presented to the subjects is large, the nerve cells in the visual cortices of the subjects respond asynchronously to the stimulus which changes over time, and the brain wave signals deviate from the sine wave, as compared to when the stress is small. Therefore, the stress caused by the sensory stimulus presented to the subjects may be evaluated by indexing the response changes of brain waves in the visual cortices regarding the stimulus which changes over time before and after the stress caused by the sensory stimulus is provided.

Further, brain wave measurement is preferably performed placing the electrodes in compliance with the International 10- 20 System. Fig. 3 is a schematic diagram illustrating the electrode arrangement for the electroencephalograph. The diagram is from the perspective of the top of the head of a viewer 1, in which the upper half of Fig. 3 is the front (nose), and the lower half of Fig. 3 is the back (occipital side), the left half of Fig. 3 is the left side (left ear), and the right half of Fig. 3 is the right side (right ear) . $A_1$, $A_2$, $O_1$, $O_2$, $P_2$, $P_3$, $P_4$, $C_2$, $C_3$, $C_4$, $F_2$, $F_3$, $F_4$, $T_3$, $T_4$, $T_5$, $T_6$, $F_2$, $F_3$, $F_4$, $F_7$, $F_8$, $Fp_1$, and $Fp_2$ represent each electrode. A1 and A2 are called the reference electrodes, and are typically attached near the earlobe which has an equivalent electrical potential of zero. The other electrodes are called the active electrodes, and detect the changes in electrical potential as compared to the reference electrode as brain wave signals. $O_1$ and $O_2$ detect brain waves in the occipital region, $P_2$, $P_3$, and $P_4$ detect in the parietal region, $C_2$, $C_3$, and $C_4$ detect in the central region, $T_3$, $T_4$, $T_5$, and $T_6$ detect in the temporal region, and $F_2$, $F_3$, $F_4$, $F_7$, $F_8$, $Fp_1$, and $Fp_2$ detect in the frontal region.

NIRS Device

The Near Infrared Spectroscopy (NIRS) device measures changes in cerebral blood flow produced in accompaniment with changes in action potential of nerve cells. The nerve cells consume oxygen during activity changes, and become temporarily oxygen-starved. In order to resolve this, blood is sent to blood vessels in the brain near these nerve cells to supply oxygen to the nerve cells. The NIRS device is a brain activity measuring method that measures changes in the amount of cerebral blood flow and the ratio of oxygenated hemoglobin and reduced hemoglobin included therein.

A light-sending and light-receiving probe is attached to the head of a subject when using the NIRS device. The light-sending probe illuminates near-infrared light toward the interior of the brain of the subject, and the near-infrared light illuminated toward the interior of the brain of the subject is reflected back to the surface of the head by the cerebral cortex, which is detected by the light-receiving probe. The oxygenated hemoglobin and the deoxygenated hemoglobin included in the cerebral blood flow has different absorption spectrums in response to the light in the near-infrared wavelength region, the near-infrared light illuminated from the light-sending probe is absorbed by the oxygenated hemoglobin and the deoxygenated hemoglobin included in the cerebral blood flow, and so the reduction in the amount of light detected by the light-receiving probe reflects the amounts of oxygenated hemoglobin and the deoxygenated hemoglobin. Therefore, the amount of cerebral blood flow in the area to which the near-infrared light passed and the ratio between the oxygenated hemoglobin and the deoxygenated hemoglobin included therein may be estimated from the changes in the amount of light from when illuminated to when detected. By measuring the changes in the amount of light over time, the temporal changes in the amount of cerebral blood flow for the area to which the light was illuminated and the ratio between the oxygenated hemoglobin and the deoxygenated hemoglobin included therein may be recorded as brain activity time-series data.

Here, if the stimulus which changes over time used is a video in which playback and stopping is repeated at fixed time intervals, the action potential of nerve cells in the visual cortices increase causing an increase in cerebral blood flow when the video is in playback mode, as compared to when the video is in stopped mode. The NIRS device detects these changes in cerebral blood flow as signal changes. When the playback and stopping of the video is reciprocally repeated at fixed time intervals, the brain responses in the visual cortices reciprocally and repeatedly rise and fall in accompaniment with the playback and stopping. That is to say, if the repeating frequency of the playback and stopping of the stimulus which changes over time is designated as f, the waveform of the detected signals measured by the NIRS device approximate the sine waveform of the frequency f.

When the stress provided to the subjects is small, the signals measured by the NIRS device from the visual cortices of the subjects synchronously respond to the stimulus which changes over time, and signals of a waveform approximating the sine wave of the repeating frequency of the stimulus which changes over time may be acquired. In contrast, when the stress provided to the subjects is large, the nerve cells in the visual cortices of the subjects respond asynchronously to the stimulus which changes over time, and the detected signals measured by the NIRS device deviate from the sine wave, as compared to when the stress is small. Therefore, the stress caused by the sensory stimulus presented to the subjects may be evaluated by indexing the response changes in cerebral blood flow for the visual cortices regarding the stimulus which changes over time before and after the stress caused by the sensory stimulus is provided.

fMRI Device

The fMRI device is a method to visualize response changes in blood flow related to brain activity using an MRI device. When using an fMRI device to measure changes in cerebral blood flow, normally brain activity is visualized by identifying the area that responded to the sensory stimulus by statistical analysis, and displaying a dissected image of this area as a color map. The device configuration of the fMRI device is different to that of the NIRS device, but such points as measuring changes in cerebral blood flow and the ability to evaluate stress provided to subjects by the sensory stimulus are the same as that of the NIRS device, and so such description is omitted here.

Brain Activity Information Acquiring Method

The brain activity information is acquired by measuring the previously described brain activity. Also, it may be acquired by evaluating the data acquired by measuring brain activity. The information acquiring method according to the present embodiment acquires information related to stress.

Stress Evaluation Method

The brain activity information acquired by the information acquiring method according to the present embodiment may be information related to stress. By acquiring information related to stress, the stress provided to biological subjects by the sensory stimulus may be evaluated. The method to evaluate the stress is performed based on the brain activity data when the stimulus which changes over time is presented before and after the sensory stimulus is presented. For example, the brain activity data in which when the stimulus which changes over time is presented before and after the stress provided by the sensory stimulus is indexed as a cross correlation. The cross correlation may be quantified by decreases in the amount of the coherence and the size of the cross correlation. The following describes details on the coherence and the cross correlation.

Coherence

The coherence in the present embodiment is acquired in the following way. First, when the data on the amount of stimulus that changes over time regarding the stimulus which changes over time is transformed by Fourier transformation, the most included frequency components are calculated. The most included frequency components are defined as the fundamental frequency ($f_0$). In the (Stimulus Which Changes over Time) section, the example given used a repeating frequency for the stimulus of 1/36 Hz, and in this case, the fundamental frequency is 1/36 Hz.

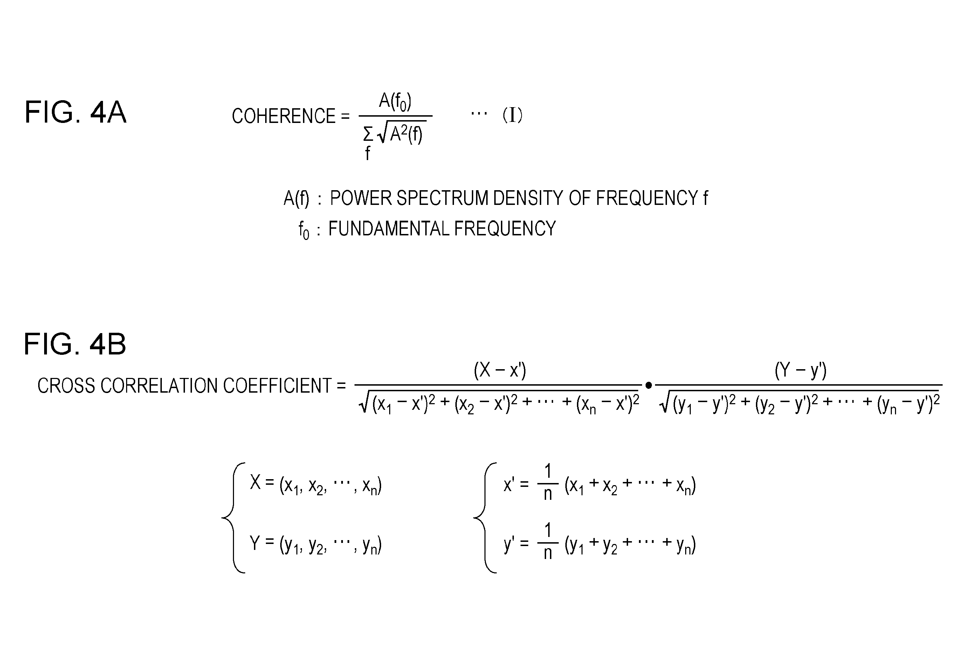

Also, the brain activity data acquired by the previously described processes (2) and (5) are converted into Fourier transformations, and the ratio accounting for the previously described fundamental frequency for all frequency components is calculated as the coherence. Specifically, the brain activity data is converted into Fourier transformations to acquire a power spectrum density. The power spectrum density is a real value calculated by multiplying a complex number acquired by converting the time- series signals (source signal) into Fourier transformations with the complex conjugate thereof. The power spectrum density is calculated for each frequency, and represents the ratio of each frequency component accounted in the source signal. When the set of power spectrum densities for each frequency is vectored, the value of the power spectrum density of the fundamental frequency components divided by the size of the vector (the square root of the sum of squares of the power spectrum density of each frequency component) is the coherence. That is to say, the coherence may be calculated using Expression (I) illustrated in Fig. 4A.

When the coherence calculated from the data acquired by process (5) has a large decrease over the coherence acquired from the data acquired by process (2), the stress provided by the sensory stimulus is large, and conversely, when the amount of decrease is small, the stress provided is also small. Therefore, video is evaluated by the information acquiring method according to the present embodiment as such: when the coherence has a small decrease, the video under evaluation does not provide stress or is not likely to provide stress; and when the coherence has a large decrease, the video under evaluation provides stress or is likely to provide stress.

The following is an example of such an evaluation. The brain activity data measured in process (2) is converted into Fourier transformations to acquire the power spectrum density and the fundamental frequency. The power spectrum density acquired for each frequency and the fundamental frequency are substituted into Expression (I) to calculate the coherence (coherence A). The brain activity data measured by process (5) is handled in the same to calculate the coherence (coherence B). Also, the difference is acquired by subtracting the value of the coherence B from the value of the coherence A. If there are multiple subjects or multiple measuring environments, the measurements are performed to acquire the differences in these coherences. Also, the average value of the differences, or the value of the smallest difference is acquired as the threshold. When larger than this threshold, it is evaluated as having stress or that the stress is large, and when smaller than this threshold, it is evaluated as not having stress, or that the stress is small. The procedure to set the threshold is not limited to only the previously described method.

By calculating the coherence in this way, stress provided to subjects by the sensory stimulus may be evaluated.

Cross Correlation

The cross correlation in the present embodiment is an index in which similarities in two sets of time-series data is quantified. For example, each set of the two sets of time-series data is vectored, the average value of all vectored elements is zero, and after normalizing the size of the vectors to produce a value of one, the inner product of these two vectors become the cross correlation. The method to acquire the cross correlation from time-series data of the previously described brain activity is described below.

(i) Data is acquired by subtracting an average value x' from all elements of the time-series data of the brain activity acquired by process (2) previously described $(x_1, x_2, x_3, ..., x_n)$.

(ii) The square root of the sum of all elements acquired by (i) squared is calculated.

(iii) The value acquired by (ii) is divided by that of (i).

(iv) The same process from (i) to (iii) is executed on the time-series data $(y_1, y_2, ..., y_n)$ of the brain activity acquired by process (5) previously described.

(v) The inner product of (iii) and (iv) is calculated.

The cross correlation acquired by performing the processes (i) through (v) is equivalent to performing a calculation of the expression illustrated in Fig. 4B by substituting the brain activity data values measured $(x_1, x_2, x_3, ..., x_n)$ and $(y_1, y_2, y_3, ..., y_n)$.

When the cross correlation is near one, the nerve cells respond synchronously to the stimulus which changes over time, and the stress provided to the subjects by the sensory stimulus is small. Conversely, when the value is near zero, the nerve cells asynchronously respond to the stimulus which changes over time, and the stress is large.

By calculating the cross correlation in this way, the stress provided to the subjects by the sensory stimulus may be evaluated.

Also, the stress evaluation method in the present embodiment may evaluate in the following way: a threshold value related to the coherence and the cross correlation is set, and when the value is the same or larger than the threshold, it is determined that stress was provided; when the value is less than the threshold value, it is determined that stress was not provided or was small.

The threshold may set manually as in the following example. A t-test is performed to determine the difference a coherence for the visual cortices on the dorsal side of the left hemisphere calculated from brain response to stimulus nearly identical to the stimulus which changes over time before the stress is provided by the sensory stimulus, and a coherence for the visual cortices on the dorsal side of the left hemisphere calculated from brain response to the stimulus which changes over time after the stress is provided by the sensory stimulus, and then a statistical test is performed to determine whether there is a significant difference between the coherence values for the two sets of data. When this is executed, a confidence interval for the average value of the difference between the two sets of data may be calculated, at the same time as being able to recognize that there is significant statistical difference between the two sets of data. By setting the upper limit of the confidence interval as the threshold, differences in coherences that deviate from the average range may be determined. Preferably, the previously described processes (1) through (6) according to the present embodiment are applied to multiple subjects beforehand, and by increasing the amount of coherence data and setting the threshold using the t-test method and such, evaluations may be performed that are not influenced by individual differences or changes in daily physical conditions of the subjects. This is an example method for setting the threshold value, and not restrictive in any way. Stimulus Which Changes over Time and Nearly Identical Stimulus

The stimulus which changes over time and the nearly identical stimulus in the present embodiment is the coherence of the stimulus which changes over time, and the absolute value of the difference between this and the coherence of the stimulus of the nearly identical value is at most 0.05. Also, it is preferable if the coherence of the stimulus which changes over time and the absolute value of the difference between this and the coherence of the stimulus of the nearly identical value is at most 0.005.

For example, the stimulus is within the difference in the previously described coherences is within the range, and there is a slight discrepancy in the time the stimulus is presented and a discrepancy between a stimulus with color and without.

The stimulus which changes over time and the nearly identical stimulus are preferably stimuli which change over time and the same stimulus.

Information Acquiring Device

The information acquiring device according to the present embodiment acquires brain activity information from biological subjects to which the sensory stimulus has been presented, and includes at least a stimulus presenting unit, a measuring unit, and a measuring control unit. The stimulus presenting unit presents a stimulus which changes over time to at least one of the brain areas in biological subjects including the visual cortices, the auditory cortices, and the vestibular cortices; a measuring unit to measure brain activity in at least one region of the brain areas; and a measuring control unit to control the measuring unit to measure the brain activity in at least one region of the brain areas when the stimulus which changes over time is given by the stimulus presenting unit. The information acquiring device according to the present embodiment acquires the brain activity information by measuring the brain activity for the brain areas while the stimulus which changes over time is given to the brain areas by the stimulus presenting unit before and after the sensory stimulus is given to the brain areas.

The stimulus which changes over time presented by the stimulus presenting unit may be the same before and after the sensory stimulus is presented, or may be the nearly identical stimulus. The nearly identical stimulus before and after the sensory stimulus is presented is in which the absolute value of the difference of the coherences between the stimulus which changes over time that is presented before the sensory stimulus and the stimulus which changes over time is at most 0.05 preferably is at most 0.005.

Further, the sensory stimulus may be presented using the stimulus presenting unit that presents the stimulus which changes over time, or may be presented using a different stimulus presenting unit. The sensory stimulus regarding the information acquiring device according to the present embodiment is preferably a visual stimulus, and also is preferably a video.

Also, the brain activity information is preferably related to stress from biological subjects for whom the information is to be acquired. By acquiring information related to stress from biological subjects, it may also be used as an evaluation device to evaluate stress. The specific process to evaluate stress has been previously described.

Also, the stimulus which changes over time preferably is a stimulus of two different types with different amounts of stimulus that is reciprocally repeated, and preferably is a video that reciprocally and repeatedly switches between movies and still images. The specific process to evaluate stress has been previously described.

Also, the measuring unit preferably measures any of the following: changes in the action potential of nerve cells in the brains of biological subjects for whom the information is to be acquired; changes in the electromagnetic field produced by the changes in the action potential; and changes in cerebral blood flow produced by the changes in the action potential.

Also, the brain areas to which the stimulus which changes over time is presented are preferably any of the visual cortices including V3A, the middle temporal area (MT), V7, and V4v.

Also, the brain activity information is preferably acquired based on the difference between the coherence value calculated using Expression (I) in Fig. 4A and the brain activity data measured before the sensory stimulus is presented to the brain area, and the coherence value calculated using Expression (I) described later and the brain activity data measure after the sensory stimulus is presented to the brain area.

The measuring unit may use the previously described nerve activity recording device, an electroencephalograph, a magnetoencephalograph, an fMRI device, a NIRS device, or a positron emission tomography device, etc.

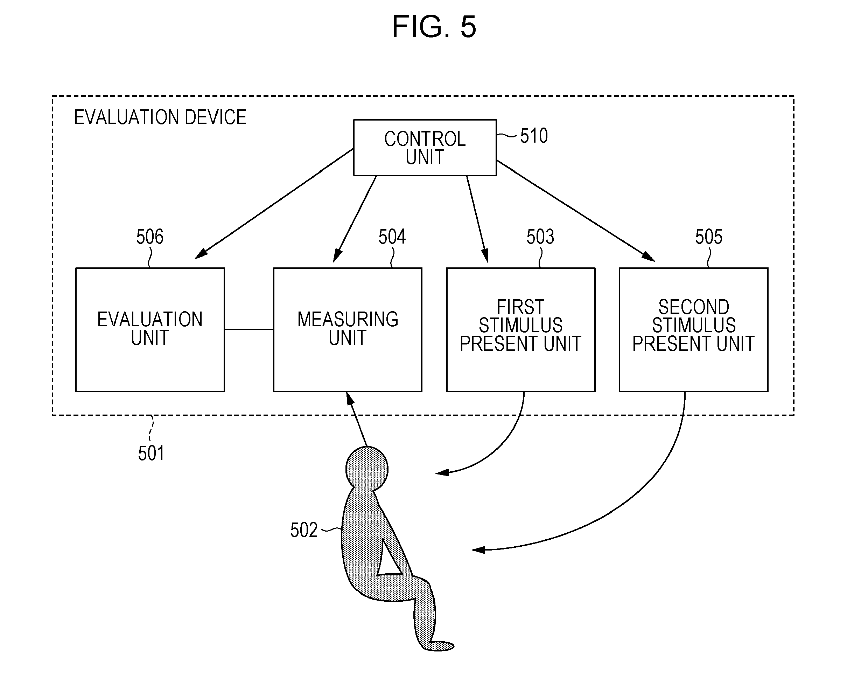

Next, as an example of the information acquiring device according to the present embodiment, an evaluation device to evaluate information related to stress will be described using Fig. 5.

An evaluation device 501 according to the present embodiment evaluates stress provided to subjects by the sensory stimulus. The evaluation device 501 includes at least a first stimulus presenting unit 503 to present the stimulus which changes over time to any of the brain areas including the visual cortices, the auditory cortices, and the vestibular cortices of a subject 502, a measuring unit 504 to measure brain activity for at least one region in the brain areas, a second stimulus presenting unit 505 to present a stimulus to the subject by the sensory stimulus, and an evaluation unit 506 to evaluate the stress based on the brain activity measured by the measuring unit.

Also, the measuring unit measures brain activity when the stimulus which changes over time is presented before the stimulus presented by the second stimulus presenting unit, and brain activity when the stimulus which changes over time is presented after the stimulus presented by the second stimulus presenting unit. Also, the evaluation unit evaluates stress based on the brain activity data measured by the measuring unit before the stimulus is presented by the second stimulus presenting unit, and the brain activity data measured by the measuring unit after the stimulus is presented by the second stimulus presenting unit. In this way, by using the stress evaluation device according to the present embodiment, stress provided to the subjects by the sensory stimulus may be evaluated by measuring brain activity in regions of brain areas which perform initial processing of sensory information, i.e. the visual cortex, auditory cortex, and vestibular cortices, in place of the brain areas such as the frontal lobe which governs executive functions. For this reason, factors other than the sensory stimulus are unlikely to influence the evaluation of stress provided by the sensory stimulus. Therefore, the evaluation device according to the present embodiment may evaluate stress provided by the sensory stimulus at a high level of accuracy as factors other than the sensory stimulus presented to the subjects are not likely to have any influence.

The specific evaluation method, for example, evaluates stress based on the difference between the coherence value calculated using Expression (I) in Fig. 4A from the brain activity data measured by the measuring unit before the sensory stimulus is presented to the brain area by the second stimulus presenting unit, and the coherence value calculated using Expression (I) described later from the brain activity data measured by the measuring unit after the sensory stimulus is presented to the brain area by the second stimulus presenting unit. The method to calculate the coherence value is as previously described.

A specific example of the method to evaluate stress using the evaluation device according to the present embodiment will now be described. First, the stimulus which changes over time is presented using the first stimulus presenting unit 503 to any brain area of the subject 502 including the visual cortices, auditory cortices, and vestibular cortices. The brain activity of at least one region in the brain area is measured by the measuring unit 504 while the stimulus which changes over time is presented. Then, stress is provided to the subject 502 by the sensory stimulus using the second stimulus presenting unit 505. Afterwards, the nearly identical stimulus to the stimulus which changes over time supplied by the first stimulus presenting unit 503 is presented to the brain area using the first stimulus presenting unit 503. The brain activity of at least one region in the brain area is measured by the measuring unit 504 while the nearly identical stimulus is presented. Then, the stress is evaluated based on the brain activity data measured when the stimulus which changes over time was presented and the brain activity data measured when the nearly identical stimulus was presented. Further, with a control unit 510, the stress evaluation device according to the present embodiment may control the first stimulus presenting unit 503, the measuring unit 504, the second stimulus presenting unit 505, and the evaluation unit 506 for evaluating stress. For example, when a video that cyclically changes is presented by the first stimulus presenting unit 503, the cyclical changes are controlled by the control unit 510. Also, the timing of measuring the brain activity may be automatically controlled by the control unit 510. Further, the evaluation device according to the present embodiment may be manually operated without using the previously described control unit 510.

Program

An example of the program according to the present embodiment is an information acquiring program configured to acquire brain activity information from biological subjects to which the sensory stimulus is presented, and executes the processes including the  previously described processes (1) through (6).

Another example of the program according to the present embodiment is an information acquiring program configured to acquire brain activity information from biological subjects to which the sensory stimulus is presented using the previously described information acquiring device to acquire the brain activity information. The information acquiring device includes a stimulus presenting unit for giving a stimulus which changes over time to at least one brain area of the biological subjects including the visual cortices, the auditory cortices, and the vestibular cortices; a measuring unit to measure brain activity in the brain areas; and a measuring control unit to control the measuring unit to measure the brain activity in the brain areas when the stimulus which changes over time is presented by the stimulus presenting unit. The program in the present example is configured to use the information acquiring device to acquire the brain activity information by executing the processes in which the brain activity is measured for at least one region in the brain areas while the stimulus which changes over time is presented to the brain areas by the stimulus presenting unit before and after the sensory stimulus is presented to  at least one of the brain areas of biological subjects including the visual cortices, the auditory cortices, and the pareto-insular vestibular cortex.

The brain activity information acquired by the information acquiring program according to the present embodiment may be information related to stress. By acquiring information related to stress, stress provided to biological subjects by the sensory stimulus may be evaluated. The specific process to evaluate stress is as described previously.

The program according to the present embodiment may be recorded on a recording medium, or may be downloaded from the Internet. The program is in a computer-readable format.

Recording Medium

An example of the recording medium according to the present embodiment is a recording medium to which the information acquiring program configured to acquire brain activity information from biological subjects to which the sensory stimulus is presented is stored, and records the information acquiring program that executes the processes including the previously described processes (1) through (6) in a computer-readable format.

Another example of the recording medium according to the present embodiment is a recording medium that records the information acquiring program configured to acquire brain activity information from biological subjects to which the sensory stimulus is presented using the previously described information acquiring device to acquire the brain activity information. The information acquiring device includes a stimulus presenting unit for giving a stimulus which changes over time to at least one brain area of the biological subjects including the visual cortices, the auditory cortices, and the vestibular cortices; a measuring unit to measure brain activity in the brain areas; and a measuring control unit to control the measuring unit to measure the brain activity in the brain areas when the stimulus which changes over time is presented by the stimulus presenting unit. The recording medium in the present example records in a computer-readable format the information acquiring program configured to use the information acquiring device to acquire the brain activity information by executing the processes in which the brain activity is measured for at least one region in the brain areas while the stimulus which changes over time is presented to the brain areas by  the stimulus presenting unit before and after the sensory stimulus is presented to at least one of the brain areas of biological subjects including the visual cortices, the auditory cortices, and the pareto-insular vestibular cortex.

The brain activity information acquired using the recording medium according to the present embodiment may be information related to stress. By acquiring information related to stress, stress provided to biological subjects by the sensory stimulus may be evaluated. The specific process to evaluate stress is as described previously. The recording medium may be a CD (CDR, CDRW, etc.), a DVD (DVDR, DVDRW, etc.), a flash memory, a hard disk, or magnetic tape, a floppy disk.

Other Embodiments

The present technology will further be described regarding another embodiment, but the present technology is not restricted thereby. According to the embodiment the sensory stimulus is described as a visual stimulus. An fMRI device is used to measure brain activity. The visual stimulus is a video (hereafter, evaluation video) that presents a feeling of shakiness to subjects (hereafter, viewers), and this kind of video may cause visually induced motion sickness in viewers. A reference video which will be described in detail later is used as the stimulus which changes over time.

The brain activity information acquired with the embodiment is data of changes in cerebral blood flow, information related to stress in included in the acquired data of changes in cerebral blood flow, and so the stress provided to viewers by the evaluation video which has been presented to the viewers may be evaluated on the basis of the information related to this stress.

According to the embodiment, the stress provided to viewers by this evaluation video is evaluated by implementing the following Implementation Procedure. Implementation Procedure

The embodiment includes the following six processes.

(1) Process to present the reference video to the viewer
(2) Process to measure cerebral blood flow of the viewer regarding process (1)
(3) Process to present the evaluation video to the viewers
(4) Process to present the reference video to the viewers
(5) Process to measure cerebral blood flow of viewers regarding process (4)
(6) Process to evaluate the stress provided to the viewers by the evaluation video on the basis of the data of cerebral blood flow measured in processes (2) and (5)

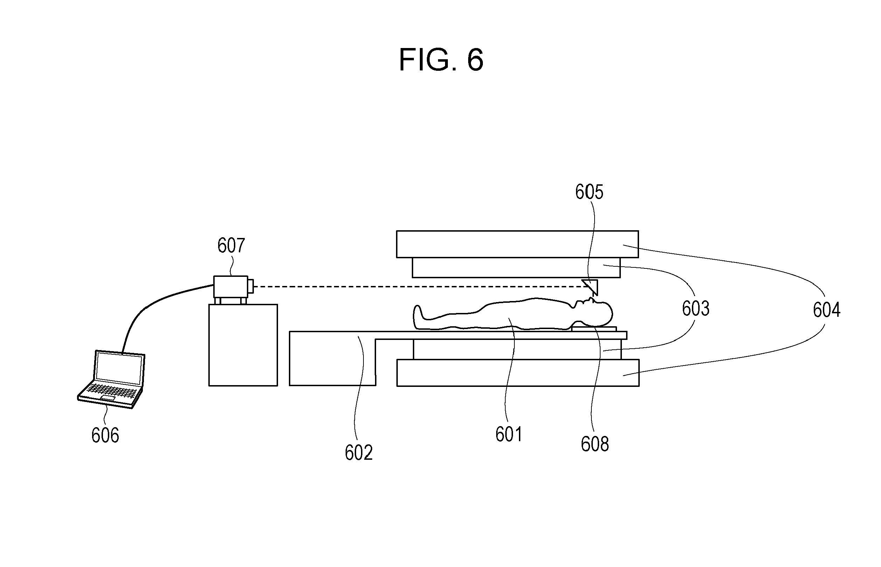

According to the embodiment, an fMRI device is used to measure the cerebral blood flow. Fig. 6 is a cross-sectional diagram of the fMRI device according to the embodiment. A viewer 601 lies on a bed 602 attached to the fMRI device, and a gradient magnetic coil 603 and a superconducting magnet 604 is arranged within a provisioned tube-shaped measuring unit. A screen 605 is provisioned near the eyes of the viewer 601 in the measuring unit, and a control computer 606 controls a video projector 607 to project the video from outside the measuring unit. A signal detection coil 608 is arranged near the occipital area of viewer, and electromagnetic signals correlating to changes in the cerebral blood flow produced from changes in action potential of nerve cells are detected. The video projector 607 is controlled by the control computer 606, and performs the display and non-display of the evaluation video, the evaluation results, and such. Next, each process will be described in detail.

(1) Process to present the reference video to the viewer

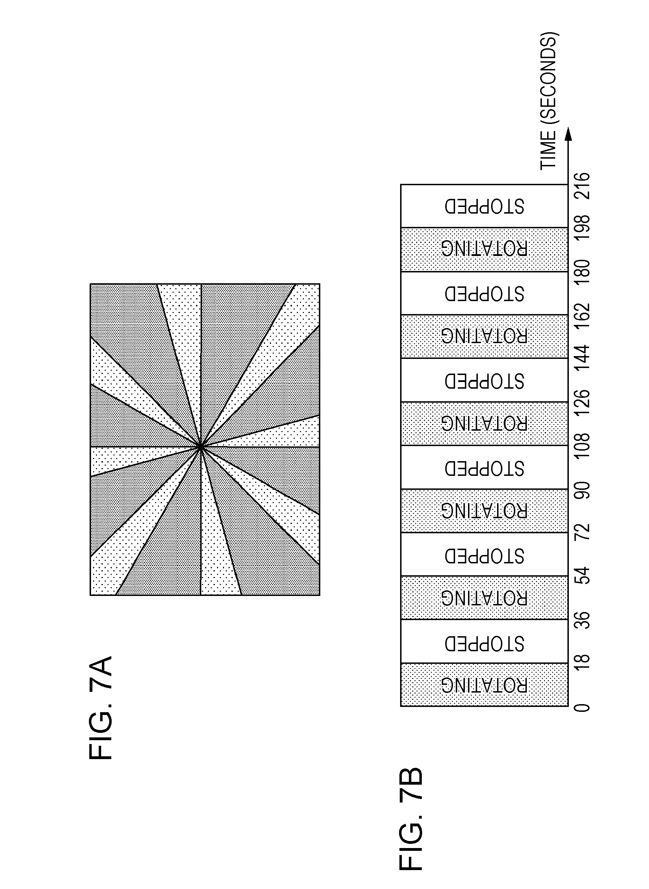

According to the embodiment, a video of a fan pattern as illustrated in Fig. 7A that reciprocally and repeatedly rotates and stops is used as the visual stimulus which changes over time (reference video). As illustrated in Fig. 7B, the fan pattern in the reference video is rotated for 18 seconds, then stopped for 18 seconds, which is repeated 6 times for a total of 216 seconds.

(2) Process to measure the brain activity in the viewer regarding process (1)

Cerebral blood flow of the viewer to which the reference video is presented is measured while process (1) is occurring. According to the embodiment, cerebral blood flow for the visual cortices responding to the visual stimulus is measured.

The data of cerebral blood flow set forth in the embodiment, that is to say, brain activity signals are acquired in the following environment. The functional brain images were acquired using a clinical use Signa Horizon 1.5-tesla MRI (manufactured by General Electric). When acquiring the functional brain images for the visual cortices, brain activity signals near the occipital lobe are measured at a high sensitivity, and so a surface coil wrapping around the occipital region is used. 22 parallel slices from the occipital pole to the frontal side in a direction nearly perpendicular to the calcarine sulcus of the subject were acquired. The repetition time (TR) is three seconds, and the voxel size is $1.5 \times 1.5 \times 4\ mm^3$.

Prior to the function brain imaging, multiple visual cortices distributed in the occipital lobe of the viewer were identified using the phase-encoding method ("Retinotopic organization in human visual cortex and the spatial precision of functional MRI", S. A. Engel, G. H. Glover, and B. A. Wandell, Cerebral Cortex, July, 1997 (2): pp. 181-192). After acquiring the functional brain images, each signal values of the function brain images for each visual cortex previously identified by the phase-encoding method is sampled as brain activity time-series data. The areas measured include the visual cortices V1 (V1d, V1v,), V2 (V2d, V2v), V3, V3A, V4 (V4d, V4v), the middle temporal area (MT), the middle superior temporal area (MST), V7, and VP area of each brain area on both the left and the right cerebral hemispheres.

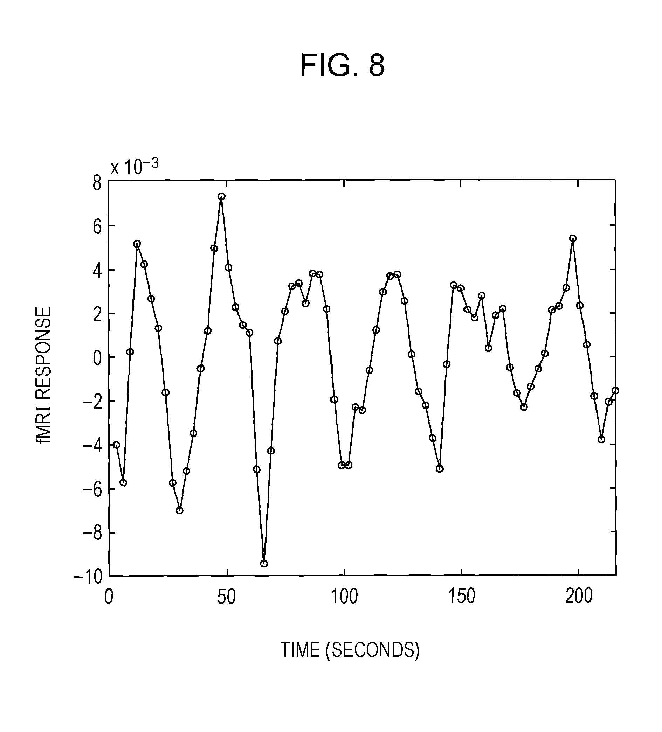

One set of the brain activity time-series data is illustrated in Fig. 8. When the movie included in the reference video is started, the nerve cells in the visual cortices respond to the movie, and brain activity signal begins to rise. After the video continues for 18 seconds, 18 seconds of a still image is presented next. While the still image is presented, the nerve cells in the visual cortices acclimate to the still image, and the brain activity signal falls. If the movie and the still image repeat six times, the brain activity signals of the visual cortices exhibit a sine wave response that repeats rising and falling six times corresponding to the video.

(3) Process to present the evaluation video to the viewer

According to the embodiment, a video causing visually induced motion sickness, which is the evaluation video, is presented to the viewer following processes (1) and (2).

(4) Process to present the reference video to the viewer

The reference video presented to the viewer in process (1) is presented to the viewer again following process (3).

(5) Process to measure brain activity of the viewer regarding process (4)

The brain activity of the subject in response to the reference video is measured while process (4) occurs. The brain areas measured are the same as for process (2) and include the visual cortices V1 (V1d, V1v,), V2 (V2d, V2v), V3, V3A, V4 (V4d, V4v), the middle temporal area (MT), the middle superior temporal area (MST), V7, and VP area of each brain area on both the left and the right cerebral hemispheres.

(6) Process to determine whether or not the video which is sensory stimulus has subjected the viewers to stress, on the basis of the amount of change in data of brain activity measured in processes (2) and (5)

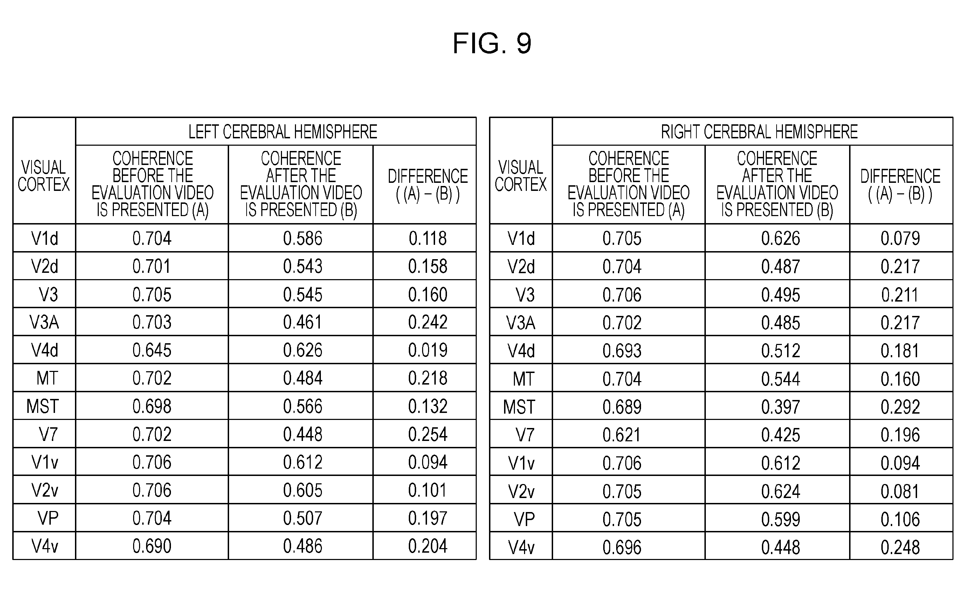

The coherence derived from the brain activity measured in processes (2) and (5) is acquired. The result is illustrated in Fig. 9.

Further, the coherence is calculated in the following way. First, the repetition frequency of the movie and the still image included in the reference video is 1/36 Hz, and this is the reference frequency. The measure brain activity data is converted into Fourier transformations to acquire the power spectrum density. The acquired power spectrum density is substituted into the expression illustrated in Fig. 4A to acquire the coherence.

Further, the brain activity time- series data includes low- frequency noise components such as noise from the fMRI device and noise from the heart beating and breathing. Here, preprocessing is performed to remove the low-frequency noise components in the brain activity time- series data to increase the accuracy of the coherence.

In this way, the coherence value (B) calculated from brain activity of the viewer after the evaluation video was presented is smaller than the coherence value (A) calculated from brain activity of the viewer before the evaluation video was presented. Therefore, the difference between (A) and (B) is calculated, and when the difference is large, the stress provided to the biological subjects by the evaluation video is large; and when the difference is small, the stress provided to the physical body by the evaluation video is small.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions. An information acquiring method configured to acquire brain activity information from biological subjects presented a sensory stimulus, in which the brain activity information is acquired by measuring the brain activity in at least one region of the brain areas of the biological subjects including the visual cortices of the cerebral cortex, the auditory cortices of the cerebral cortex, and the vestibular cortices of the cerebral cortex; while the stimulus which changes over time is presented to the brain areas by stimulus present means (503, 505) before and after the sensory stimulus is presented to the brain areas.

**Claims**

1. An information acquiring method to acquire brain activity information from biological subjects presented a sensory stimulus, the method comprising:

   (1) a process (S101) to present a stimulus which changes over time to at least one brain area of the biological subjects including the visual cortices of the cerebral cortex, the auditory cortices of the cerebral cortex, and the vestibular cortices of the cerebral cortex;
   (2) a process (S102) to measure brain activity in at least one region of the brain areas while the stimulus which changes over time is presented;
   (3) a process (S103) to present said sensory stimulus after process (2) (S102);
   (4) a process (S104) to present a nearly identical stimulus to the stimulus which changes over time to the brain areas after process (3) (S103);
   (5) a process (S105) to measure brain activity for at least one region of the brain areas while the nearly identical stimulus is presented; and
   (6) a process (S106) to acquire the brain activity information on the basis of the brain activity data measured in process (2) (S102) and the brain activity data measured in process (5) (S105).

2. The information acquiring method according to claim 1, wherein the sensory stimulus is a visual stimulus.

3. The information acquiring method according to claim 1or 2, wherein the brain activity information is information related to stress in the biological subjects.

4. The information acquiring method according to any one of claims 1 through 3, wherein the stimulus which changes over time is a video that reciprocally repeats between a movie and a still image.

5. The information acquiring method according to any one of claims 1 through 4, wherein the brain areas are any of the following including the visual cortices of the cerebral cortex V3A, the middle temporal area (MT), V7, and V4v.

6. The information acquiring method according to any one of claims 1 through 5, wherein the process (6) (S106) acquires the brain activity information on the basis of the difference between a coherence value calculated from the brain activity data measure in the process (2) (S102) using expression (I), and a coherence value calculated from the brain activity data measure in the process (5) (S105) using expression (I),

$$\mathrm{Coherence} = \frac{\mathrm{A}(f_0)}{\sum_f \sqrt{\mathrm{A}^2(f)}} \qquad (\mathrm{I})$$

where A(f) represents the power spectrum density of the frequency f, and $f_0$ represents the fundamental frequency.

7. An information acquiring device (501) to acquire brain activity information from biological subjects presented with a sensory stimulus, the device comprising:

   stimulus present means (503, 505) to present a stimulus which changes over time to at least one brain area of the biological subjects including the visual cortices of the cerebral cortex, the auditory cortices of the cerebral cortex, and the vestibular cortices of the cerebral cortex;
   measuring means (504) to measure brain activity in at least one region of the brain areas; and
   measuring control means (510) to control the measuring means (504) to measure the brain activity in the brain areas when the stimulus which changes over time is given to the brain areas by the stimulus present means (503, 505); wherein
   the brain activity information is acquired by measuring the brain activity in at least one region of the brain areas while the stimulus which changes over time is presented to the brain areas by the stimulus present means (503, 505) before and after the sensory stimulus is presented to the brain areas.

8. The information acquiring device (501) according to claim7, wherein the sensory stimulus is a visual stimulus.

9. The information acquiring device (501) according to claim 7 or 8, in which the brain activity information is information related to stress in the biological subjects.

10. The information acquiring device (501) according to any one of claims 7 through 9, wherein the stimulus which changes over time is a video that reciprocally repeats between a movie and a still image.

11. The information acquiring device (501) according to any one of claims 7 through 10, wherein the brain areas are any of the following including the visual cortices of the cerebral cortex V3A, the middle temporal area (MT), V7, and V4v.

12. The information acquiring device (501) according to any one of claims 7 through 11, wherein the brain activity information is acquired on the basis of the difference between a coherence value calculated from the brain activity data measured before giving the sensory stimulus using expression (I), and a coherence value calculated from the brain activity data measure after giving the sensory stimulus using expression (I),

$$\text{Coherence} = \frac{A(f_0)}{\sum_f \sqrt{A^2(f)}} \quad (\text{I})$$

where A(f) represents the power spectrum density of the frequency f, and $f_0$ represents the fundamental frequency.

13. An information acquiring program to acquire brain activity information from biological subjects presented a sensory stimulus by executing the following processes:

(1) a process (S101) to present a stimulus which changes over time to at least one brain area of the biological subjects including the visual cortices of the cerebral cortex, the auditory cortices of the cerebral cortex, and the vestibular cortices of the cerebral cortex;
(2) a process (S102) to measure brain activity in at least one region of the brain areas while the stimulus which changes over time is presented;
(3) a process (S103) to present said sensory stimulus after process (2) (S102);
(4) a process (S104) to present a nearly identical stimulus to the stimulus which changes over time to the brain areas after process (3) (S103);
(5) a process (S105) to measure brain activity for at least one region of the brain areas while the nearly identical stimulus is presented; and
(6) a process (S106) to acquire the brain activity information on the basis of the brain activity data measured in process (2) (S102) and the brain activity data measured in process (5) (S105).

14. An information acquiring program to execute the processes to acquire brain activity information by measuring the brain activity in at least one region of brain areas while stimulus, which changes over time, is presented to the brain areas by stimulus present means (503, 505) before and after the sensory stimulus is presented to the brain areas, using an information acquiring device (501) which includes
stimulus present means (503, 505) to present a stimulus which changes over time to at least one brain area of the biological subjects including the visual cortices, the auditory cortices, and the vestibular cortices,
measuring means (504) to measure brain activity in at least one region of the brain areas, and
measuring control means (510) to control the measuring means (504) to measure the brain activity in the brain areas when the stimulus which changes over time is given by the stimulus present means (503, 505).

# FIG. 1

| START TO PRESENT STIMULUS | ~S101 |

$\downarrow$

| MEASURE BRAIN ACTIVITY | ~S102 |

$\downarrow$

| START TO PRESENT SENSORY STIMULUS | ~S103 |

$\downarrow$

| START TO PRESENT NEARLY IDENTICAL STIMULUS | ~S104 |

$\downarrow$

| MEASURE BRAIN ACTIVITY | ~S105 |

$\downarrow$

| START TO ACQUIRE BRAIN ACTIVITY INFORMATION | ~S106 |

FIG. 2A

FIG. 2B

EP 2 649 935 A2

# FIG. 3

FRONT SIDE
(NOSE)

Fp₁    Fp₂

F₇    F₃    F₂    F₄    F₈

LEFT SIDE        A₁    T₃    C₂    T₄    A₂        RIGHT SIDE
(LEFT EAR)                  C₃        C₄                  (RIGHT EAR)

P₃    P₂    P₄    T₆

T₅

O₁    O₂

OCCIPITAL SIDE

# FIG. 4A

$$\text{COHERENCE} = \frac{A(f_0)}{\sum_f \sqrt{A^2(f)}} \quad \cdots \quad (\text{I})$$

A(f) : POWER SPECTRUM DENSITY OF FREQUENCY f

$f_0$ : FUNDAMENTAL FREQUENCY

# FIG. 4B

$$\text{CROSS CORRELATION COEFFICIENT} = \frac{(X - x')}{\sqrt{(x_1 - x')^2 + (x_2 - x')^2 + \cdots + (x_n - x')^2}} \bullet \frac{(Y - y')}{\sqrt{(y_1 - y')^2 + (y_2 - y')^2 + \cdots + (y_n - y')^2}}$$

$$\begin{cases} X = (x_1, x_2, \cdots, x_n) \\ Y = (y_1, y_2, \cdots, y_n) \end{cases} \qquad \begin{cases} x' = \dfrac{1}{n}(x_1 + x_2 + \cdots + x_n) \\ y' = \dfrac{1}{n}(y_1 + y_2 + \cdots + y_n) \end{cases}$$

EP 2 649 935 A2

# FIG. 5

EVALUATION DEVICE

CONTROL UNIT — 510

506 EVALUATION UNIT

504 MEASURING UNIT

503 FIRST STIMULUS PRESENT UNIT

505 SECOND STIMULUS PRESENT UNIT

501

502

EP 2 649 935 A2

FIG. 6

# FIG. 7A

# FIG. 7B

| | ROTATING | STOPPED | ROTATING | STOPPED | ROTATING | STOPPED | ROTATING | STOPPED | ROTATING | STOPPED | ROTATING | STOPPED | |
|---|---|---|---|---|---|---|---|---|---|---|---|---|---|
| 0 | 18 | 36 | 54 | 72 | 90 | 108 | 126 | 144 | 162 | 180 | 198 | 216 | TIME (SECONDS) |

# FIG. 8

# FIG. 9

| VISUAL CORTEX | LEFT CEREBRAL HEMISPHERE | | | VISUAL CORTEX | RIGHT CEREBRAL HEMISPHERE | | |
|---|---|---|---|---|---|---|---|
| | COHERENCE BEFORE THE EVALUATION VIDEO IS PRESENTED (A) | COHERENCE AFTER THE EVALUATION VIDEO IS PRESENTED (B) | DIFFERENCE ( (A) − (B) ) | | COHERENCE BEFORE THE EVALUATION VIDEO IS PRESENTED (A) | COHERENCE AFTER THE EVALUATION VIDEO IS PRESENTED (B) | DIFFERENCE ( (A) − (B) ) |
| V1d | 0.704 | 0.586 | 0.118 | V1d | 0.705 | 0.626 | 0.079 |
| V2d | 0.701 | 0.543 | 0.158 | V2d | 0.704 | 0.487 | 0.217 |
| V3 | 0.705 | 0.545 | 0.160 | V3 | 0.706 | 0.495 | 0.211 |
| V3A | 0.703 | 0.461 | 0.242 | V3A | 0.702 | 0.485 | 0.217 |
| V4d | 0.645 | 0.626 | 0.019 | V4d | 0.693 | 0.512 | 0.181 |
| MT | 0.702 | 0.484 | 0.218 | MT | 0.704 | 0.544 | 0.160 |
| MST | 0.698 | 0.566 | 0.132 | MST | 0.689 | 0.397 | 0.292 |
| V7 | 0.702 | 0.448 | 0.254 | V7 | 0.621 | 0.425 | 0.196 |
| V1v | 0.706 | 0.612 | 0.094 | V1v | 0.706 | 0.612 | 0.094 |
| V2v | 0.706 | 0.605 | 0.101 | V2v | 0.705 | 0.624 | 0.081 |
| VP | 0.704 | 0.507 | 0.197 | VP | 0.705 | 0.599 | 0.106 |
| V4v | 0.690 | 0.486 | 0.204 | V4v | 0.696 | 0.448 | 0.248 |

EP 2 649 935 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **IIJIMA ; ATSUHIKO et al.** Cerebral blood flow and subjective score during visually induced motion sickness. *23rd BPES Symposium on Biological and Physiological Engineering,* 2008, 11-12

- **S. A. ENGEL ; G. H. GLOVER ; B. A. WANDELL.** Retinotopic organization in human visual cortex and the spatial precision of functional MRI. *Cerebral Cortex,* July 1997, 181-192